# EUROPEAN PATENT SPECIFICATION

(11) **EP 2 978 847 B1**
(45) Date of publication and mention of the grant of the patent: **15.02.2017**
(21) Application number: 14719866.7
(22) Date of filing: 26.03.2014
(51) Int. Cl.: C12N 15/113

(54) **HAMMERHEAD RIBOZYMES TARGETING MIR-21**
GEGEN MIR-21 GERICHTETE HAMMERKOPF-RIBOZYME
RIBOZYMES EN TÊTE DE MARTEAU CIBLANT MIR-21

(30) Priority: 27.03.2013 PL 40334113
(43) Date of publication of application: 03.02.2016
(73) Proprietor: Instytut Chemii Bioorganicznej Polskiej Akademii Nauk, 61-704 Poznan (PL)
(72) Inventor: NASKRET-BARCISZEWSKA, Miroslawa, 60-334 Poznan (PL); BELTER, Agnieszka, 64-761 Krzyz (PL); ROLLE, Katarzyna, 62-023 Kamionki (PL); PIWECKA, Monika, 60-687 Poznan (PL); SOSINSKA, Patrycja, 62-020 Swarzedz (PL); FEDORUK-WYSZOMIRSKA, Agnieszka, 61-609 Poznan (PL)
(74) Representative: Grzelak, Anna
(86) International application number: PCT/IB2014/060188
(87) International publication number: WO 2014/155320

(56) References cited:
- HEMANT SURYAWANSHI ET AL: "Modulation of microRNA function by synthetic ribozymes", MOLECULAR BIOSYSTEMS, vol. 6, no. 10, 1 January 2010 (2010-01-01), page 1807, XP055127691, ISSN: 1742-206X, DOI: 10.1039/c0mb00010h cited in the application
- MARTA MAGDALENA GABRYELSKA ET AL: "Prediction of hammerhead ribozyme intracellular activity with the catalytic core fingerprint", BIOCHEMICAL JOURNAL, vol. 6, no. 3, 19 February 2013 (2013-02-19), pages 2060-451, XP055128347, ISSN: 0264-6021, DOI: 10.1261/rna.631207 cited in the application
- HEMANT SURYAWANSHI ET AL: "Antagonism of microRNA Function in Zebrafish Embryos by Using Locked Nucleic Acid Enzymes (LNAzymes)", CHEMBIOCHEM, vol. 13, no. 4, 7 February 2012 (2012-02-07), pages 584-589, XP055127729, ISSN: 1439-4227, DOI: 10.1002/cbic.201100789
- M. FUKUDA ET AL: "A strategy for developing a hammerhead ribozyme for selective RNA cleavage depending on substitutional RNA editing", RNA, vol. 18, no. 9, 13 July 2012 (2012-07-13), pages 1735-1744, XP055128346, ISSN: 1355-8382, DOI: 10.1261/rna.033399.112
- A. R. FERRE-D'AMARE ET AL: "Small Self-cleaving Ribozymes", COLD SPRING HARBOR PERSPECTIVES IN BIOLOGY, vol. 2, no. 10, 15 September 2010 (2010-09-15), pages a003574-a003574, XP055128414, DOI: 10.1101/cshperspect.a003574 cited in the application

### SEQUENCE LISTING

<110> Instytut Chemii Bioorganicznej PAN
<120> Hammerhead ribozymes
<130> PZ/1904/AGR/PCT
<160> 6
<170> PatentIn version 3.5
<210> 1
   <211> 22
   <212> RNA
   <213> artificial
<220>
   <223> CORE of miR21hammerhead
<400> 1
   cugaugaggc cgaaaggccg aa 22
<210> 2
   <211> 34
   <212> RNA
   <213> artificial
<220>
   <223> miR21rz1
<400> 2
   cagucucuga ugaggccgaa aggccgaaau aagc 34
<210> 3
   <211> 34
   <212> RNA
   <213> artificial
<220>
   <223> miR21rz2
<400> 3
   ccaugacuga ugaggccgaa aggccgaaau ucaa 34
<210> 4
   <211> 34
   <212> RNA
   <213> artificial
<220>
   <223> miR21rz3
<400> 4
   cccauccuga ugaggccgaa aggccgaaac uggu 34
<210> 5
   <211> 38
   <212> RNA
   <213> artificial
<220>
   <223> hammerhead ribozyme of the state of art
<400> 5
   ucagucucug augaguccgu gaggacgaaa uaagcuac 38
<210> 6
   <211> 34
   <212> RNA
   <213> artificial
<220>
   <223> miR21rz1-mut ribozyme
<400> 6
   cagucucuga ugaggccgaa aggccgaaau aagc 34

## Description

### TECHNICAL FIELD

The object of the invention are the hammerhead ribozymes that specifically and efficiently cleave miR-21 RNA and/or its precursors (pri-miR21 and pre-miR-21), the composition and the therapeutic agent comprising them, their use for the downregulation of cellular miR-21 in therapy of cancers, preferably brain tumors and the method of cleavage of miR-21 and/or its precursors.

### BACKGROUND ART

Gliomas are the most common type among cancers of the central nervous system. Glioblastoma multiforme (GBM) represents more than 50% of all gliomas and is the most malignant (grade IV according to WHO malignancy grade) type from all the primary brain tumors. GBM is characterized by infiltrative growth pattern, abundant vascularization, rapid proliferation and aggressive clinical course. Moreover, because of its localization and substantial resistance to conventional therapies, survival prognosis are very poor.

Invariably, for many years the standard treatment in gliomas remains surgical resection accompanied with radio-and chemotherapy. Maximum cytoreduction (>98% of the tumor) prolongs survival for as long as 9-12 months. It also improves subject's response to radio- and chemotherapy. Very often due to tumor localization and its infiltrative character, the surgical intervention is not possible. Radiotherapy (RT) is usually implemented as the first adjuvant therapy after tumor resection. The standard treatment in chemotherapy is temozolomide (TMZ) and gliadel (Westphal M et al., Neurooncol 5, 79-88 (2003); Stupp R et al., N Engl J Med. 352, 987-996 (2005). In case of recurrence, faster and more aggressive growth of the tumor, as well as its enhanced resistance to treatment is observed among the patients treated with TMZ and RT.

In recent years, there was a significant progress in understanding the molecular basis of GBM. Many therapeutic targets and many potential therapeutic agents have been identified. Most of the new therapeutic approaches are focused on small-molecule inhibitors, monoclonal antibodies and peptides vaccines, used for the regulation of cellular pathways crucial for tumor development, angiogenesis and elimination of tumor cells drug resistance. Despite the good prognosis for these approaches, the majority of them have been rejected during clinical trials. Gliomas are one of the most difficult to treat tumors with the worst prognosis and average survival time of less than one year.

In the absence of effective treatments for gliomas and their resistance to conventional therapies, the challenge is to study new therapeutic targets and new approaches to the treatment of GBM.

MicroRNAs (miRNAs) are small (~22 nt) non-coding regulatory RNA molecules. They play a critical role in many cellular processes, such as: growth, differentiation, development, cell division, apoptosis, cellular signalization and gene expression. In recent years they have been associated in the progression of various cancers and proposed as a novel targets for anticancer therapies. miRNA targets, degrades or translationally represses, mRNAs. They have the capacity to regulate a large number of target mRNAs involved in cellular processes such as developmental timing, differentiation, cell proliferation, cancer or neurodegenerative disorders and apoptosis. It has been estimated that miRNAs control the expression of more than 30% of all protein encoding genes, including oncogenes and suppressor proteins (Bartel DP, Cell 116, 281-297 (2004); Esquela-Kerscher A et al., Nat Rev Cancer 6, 259-269 (2006)).

It has been estimated that changes in the miRNAs expression profile underlie more than 390 diseases (). The largest group among them is cancers. miRNAs underlie numerous neurological and psychiatric problems including brain tumors._For many of them specific miRNA expression profiles have been identified, what significantly increased the possibilities for their diagnosis and prognosis. They also indicate new potential therapeutic targets. The miRNA profile in glial tumors differs significantly from the one in healthy nerve cells. Furthermore, as in the case of a healthy brain during development, in the tumor at different stages the miRNA profile is subject to dynamic changes. The miRNA, whose levels were significantly changed in the glioblastoma multiforme cells compared to healthy cells have been identified. Studies by the inventors show that one of the miRNAs with the highest increase in expression levels in glial tumor cells is miR-21. The correlation between the level of miR-21 and the expression of its potential targets (mRNAs recognized by the miRNA) suggests, that this miRNA is directly involved in glial tumor development and could be considered as a good diagnostic marker and a potential therapeutic target for this type of tumors (Nikaki A et al., Expert Opin Investig Drugs 21, 1475-1488 (2012)).

miR-21 is the first discovered mammalian miRNA (Lagos-Quintana M et al., Science 294, 853-858 (2001)). The coding sequence of miR-21 is localized in the long arm of chromosome 17 (17q23.2, 55273409-55273480), within the gene encoding TMEM 49 (transmembrane protein 49). The *MIR21* gene is located in the intron of that gene, has its own promoter and its transcription takes place independently from the *TMEM49* transcription process. The product of the *MIR21* gene is a pri-miR-21 transcript with the length of 3433 nt, which is processed first into pre-miR-21 (72 nt), and finally to the mature miR-21 (22 nt).

The miR-21 content in the clinical specimens of brain glial tumors and cell lines derived from GBM is significantly higher in comparison to the levels of this miRNA in healthy cells with cerebral origin (Conti A et al., J Neurooncol 93, 325-332 (2009); Chan JA et al., Cancer Res 65, 6029-6033 (2005); Ciafre SA et al., Biochem Biophys Res Commun 334, 1351-1358 (2005)). Additionally, its expression levels correlate well with the tumor's malignancy and it is significantly higher in patients with a grade III tumor, compared to the patients with a grade II tumor. The downregulation of the miR-21 level is observed among patients treated with chemo- and radiotherapy. Precise diagnosis of glial tumors, determination of the stage of the disease, prediction of patients survival, selection of suitable treatment and monitoring the course of treatment is possible based on the profile of miR-21 in postoperative tissue, as well as cerebrospinal fluid and blood serum.

The functional analysis of miR-21 proves, that this miRNA is crucial in the carcinogenesis process and its downregulation or inhibition of its functionalities can restrain or stop the progress of the disease and sensitize the chemo- or radiotherapy-resistant cells to standard treatment. Currently, more than 170 potential targets for miR-21, associated with 9 cellular signaling pathways involved in carcinogenesis processes have been identified. Some of the proposed targets have been experimentally verified. For example, it has been shown that miR-21 downregulates the expression of the proteins involved in regulation of apoptosis (PDCD4, MTAP, SOX5) and chemotherapy resistance (LRRIP1). It was demonstrated that downregulation of miR-21 in cell lines results in inhibition of cellular proliferation, enhanced apoptosis, decrease in cell invasiveness (Zhou X et al., Lab Invest 90, 144-155 (2010); Li Y et al., Brain res 1286, 13-18 (2009); Corsten MF et al., Cancer Res 67,8994-9000 (2007) and in a mouse model it slows tumor growth and decreases the number of metastases (Gaur AB, Neuro Oncol 13, 580-590 (2011)). It confirms that miR-21 may be a good target for brain tumor therapy (Li Y et al., Brain Res 1286, 13-18 (2009)).

So far, the possibility of employing a few molecular tools targeting miR-21 has been analyzed (i.e. small molecule inhibitors) (Gumireddy K et al., Angew Chem Int Ed Engl 47, 7482-7484 (2008)). However, the disadvantages of the proposed tools as therapeutics are: poorly understood mechanism of action, low specificity of the compounds, potential additional inhibition of other pre-miRNA and low therapeutic index. The abovementioned studies did not directly relate to glial tumors.

Anti-miR-21 antisense oligonucleotides (AS-ON) have been tested in glial tumors (Kurreck J, Eur J Biochem 270, 1628-1644 (2003). The AS-ONs used in the context of miR-21 and glioblastoma were used in basic research, to verify and identify potential target sequences for the miRNA and to investigate the effects of miR-21 inhibition. However AS-ONs have many disadvantages such as: short half-life in serum, susceptibility to degradation by endo- and exonucleases (Campbell JM et al., J Biochem Biophys Methods 20, 259-267 (1990)), low specificity of the AS-ONs (so-called off-target effect), lower stability of AS-ON:RNA complexes, hampered transport of the AS-ONs through cell membranes and blood-brain barrier, the necessity of using a carrier, immunological response induction by the synthetic AS-ONs containing unmetylated CpG dinucleotides and dependency of the AS-ON:miRNA heteroduplex degradation on the endogenous protein machinery, mainly RNase H.

The invention relates to hammerhead ribozymes targeting miR-21 and its precursors. They are the most well-known and the smallest catalytic RNAs (Ferre-D'Amara AR et al. Cold Spring Harbor Perspectives in Biology 2, a003574 (2010)). There were found in viroids and viral satellite RNAs. During viroid replication, hammerheads act in *cis-,* self-cleaving the RNA in which they are embedded through a single turnover mechanism. Their activity is due to the catalytic core, their specificity due to the ribozyme arms complementarity to the sequences of the substrate flanking the cleavage site. A ribozyme in a complex with its substrate forms three helical segments (I, II and III) called the arms, which surround the highly conservative catalytic core sequence. The helices I and III are formed due to hybridization of the ribozyme with complementary regions in the substrate, while the II helix forms the catalytic core.

Ribozymes catalyse a sequence-specific hydrolysis of RNAs containing a 5'-NUH-3' type trinucleotide, wherein N means any nucleotide, U means uridine and H means adenosine, cytosine or uridine, which is not linked by hydrogen bonds. The efficiency of the hydrolysis of the bonds depends mainly on the target RNA sequence (Sun LQ et al., Pharmacol Rev 52, 325-347 (2000)). Other factors determining their activity, together with specificity, are the length and composition of the ribozyme arms surrounding the cleavage site. The arms ensure stable bonding of the ribozyme to the substrate and also facilitate its release from the cleavage products, such that the ribozyme becomes available for subsequent substrate molecules. Elongation of the arms may be associated with a decrease in hydrolysis efficiency; while their shortening may be associated with a decrease in ribozyme specificity.

The hammerhead ribozymes exhibit a secondary structure containing three stems that are formed between a highly conserved catalytic domain and the substrate RNA (Ruffner *et al.* 1989). The cleavage reaction, which does not require other cellular components, occurs downstream of a GUH motif (the trinucleotide preceding the self-cleavage site in most natural hammerheads, where H represents any nucleotide except G) within the RNA substrate. The'hammerhead' ribozyme requires not only accessibility to the target RNA motif, but also the formation of a proper structure for catalytic activity, thus ensuring high specificity (Hertel KJ et al., Biochemistry 33, 3374-3385 (1994); Kurreck J et al., J Biol Chem 277, 7099-7107 (2002)).

The optimal length of the hybridizing arms is 6-10 nucleotides (Scalon KJ, Curr Pharma Biotech 5, 415-420 (2004)). The inventors have shown in their studies that the efficiency of the ribozyme catalyzed cleavage also largely depends on the secondary structure of the RNA substrate.

From Suryawanshi H et al. Mol Biosystem 6, 1807-1809 (2010) a hammerhead ribozyme and its modified, nuclease-resistant variant, complementary to pre-miR-21 and miR-21 are known. Its catalytic activity was shown at *in vitro* conditions with respect to pre-miR-21, but not to miR-21. The observed effects of the use of ribozymes in cell culture were the following: decrease in the pool of endogenous miR-21, increased expression of PDCD4 protein (miR-21 target protein) and reduced cell survival. Ribozymes described in the above reference were:
The wild-type form, with the following sequence: 5'-UCAGUCUCUGAUGAGUCCGUGAGGACGAAAUAAGCUAC-3' (SEQ ID No 5)
The chemically modified form: the nucleotide sequence was as for the wild-type form, but nucleotides carried chemical modifications, which determined nuclease-resistance.

It has not been demonstrated however that the known ribozymes cleave mature miR-21 as well as pre-miR-21. Based on the complementarity of the arms sequence to miR-21 and on the activity against pre-miR-21 of the ribozymes described in Suryawanshi H et al., Mol BioSyst 6, 1807-1809 (2010) it can only be theorized that the ribozyme may be active against miR-21 as well. Also, the cell-based tests do not prove an activity of the proposed ribozymes against miR-21. The observed cellular effects may be a result of cleavage of pre-miR-21 only.

The hydrolytic activity of the ribozymes at *in vitro* conditions have been only shown at 25mM MgCl₂ (unphysiological concentration). It has not been demonstrated whether the designed ribozymes exhibit an activity at physiological concentrations of magnesium ions (i.e. about 1 mM). Moreover, the delivery of such ribozyme into the cell requires a carrier, which makes the potential therapeutic application more difficult.

So far, no ribozymes showing higher activity against pre-miR-21 and miR-21 have been described. The activity of the ribozymes can be modulated with an introduction of changes in the ribozyme sequence (eg. in the catalytic core) or in the length of the substrate-recognizing arms. These changes can increase, decrease or even abolish the ribozyme activity. For example, an introduction of three nucleotide substitutions within the 22 nucleotides-long catalytic core results in total inactivation of ribozyme activity. In the most cases there is no simple way to predict what sequence change may cause a decrease or an increase in the ribozyme activity. Each new sequence requires experimental evaluation of the ribozymes activity and efficacy (Gabryelska MM et al., Biochem J, 2013).

### DISCLOSURE OF THE INVENTION

In the light of the described state of art, the aim of the present invention is to overcome the indicated disadvantages and to provide new improved hammerhead ribozymes that specifically and efficiently cleave miR-21 and/or its precursors, while their properties enable their use in reducing the miR-21 pool in the treatment of brain tumors. The aim of the present invention is also to provide new uses of the improved ribozymes that efficiently cleave miR-21 and/or its precursors in the treatment of diseases with elevated cellular miRNA content for miR-21 and/or its precursors. The aim of the invention is also to provide a composition and therapeutic agents for the treatment of diseases with elevated cellular miRNA content for miR-21 and/or its precursors.

The invention provides the hammerhead ribozymes, specifically and efficiently cleaving miR-21 and/or its precursors, *in vitro* at physiological concentration of Mg²⁺ ions, and *in vivo* in glioma cells. Moreover, the ribozymes being the object of the invention, unlike the solutions present in the state of art, consist of natural nucleotides only, without any chemical modifications, which allows to exclude the possibility of cellular response to modified nucleotides.

The disclosure describes a hammerhead ribozyme directed against the sequence of miR-21 and/or its precursors, having the ability to specifically cleave miR-21 and/or its precursors, wherein it has a catalytic core with a sequence as shown in SEQ ID No 1, and wherein the ribozyme contains arms at both sides, preferably of 6 nucleotides in length, with sequences complementary to a region within the miR-21 and/or its precursors.

The object of the invention is a ribozyme directed against the sequence of miR-21 and/or miR-21 precursors, which has a sequence as shown in SEQ ID NO: 2 with a catalytic core with a sequence as shown in SEQ ID No 1, and having the ability to specifically cleave miR-21 and/or its precursors. The invention also relates to a ribozyme directed against the sequence of miR-21 precursors, having a sequence as shown in SEQ ID No 3, with a catalytic core with a sequence as shown in SEQ ID No 1, and having the ability to specifically cleave miR-21 and/or its precursors.

The invention also relates to a ribozyme directed against the sequence of miR-21 precursors, having a sequence as shown in SEQ ID No 4, with a catalytic core with a sequence as shown in SEQ ID No 1, and having the ability to specifically cleave miR-21 and/or its precursors.

Moreover, the invention relates to a composition, comprising at least one ribozyme according to the invention or a mixture thereof. Such composition comprising ribozymes according to the invention, preferably contains a carrier improving stability of nucleic acids or facilitating the transport of ribozymes through cell membranes. The composition preferably contains a carrier, which is Lipofectamine, for example Lipofectamine 2000.

The invention also relates to a therapeutic agent, comprising as an active agent at least one ribozyme according to the invention or a mixture thereof. The therapeutic agent preferably also contains a different component for inhibiting tumor cells growth for simultaneous or subsequent use in an anti-cancer therapy. The preferred component for inhibiting tumor cells growth is temozolomide or gliadel, and the cancer is a brain tumor, preferably a brain glioma more preferably glioblastoma multiforme.
The ribozymes, the composition and the therapeutic agent according to the invention may be used in therapy of various cancers, e.g. brain tumors, more preferably brain glioma in particular glioblastoma multiforme.

The invention also relates to the ribozyme according to the invention or a mixture thereof, or a composition of the invention or a therapeutic agent of the invention for use in the treatment of diseases with elevated cellular miRNA pool for miR-21 and/or miR-21 precursors especially for the treatment of cancer with elevated cellular miRNA pool for miR-21 and/or miR-21 precursors, preferably the cancer is a brain tumor, more preferably brain glioma, even more preferably glioblastoma multiforme.

The disclosure describes a use of the ribozymes according to the invention or a mixture thereof, the composition according to the invention, the therapeutic agent according to the invention for the manufacture of a medicament for the treatment of diseases with elevated cellular miRNA pool for miR-21 and/or its precursors. Preferably, the disease with elevated cellular miRNA content for miR-21 and/or its precursors is cancer, preferably the cancer is a brain tumor, preferably brain glioma, more preferably glioblastoma multiforme. Such use allows selective destruction of cancer cells. Such a therapeutic agent or medicament can be used for the downregulation of the miR-21cellular pool, in brain tumor therapy, particularly in therapy of glioblastoma multiforme and other diseases with an elevated pool of miR-21 and/or its precursors.

The invention also relates to a method of selective cleavage of miR-21 and/or its precursors, comprising the step of complex formation of miR-21 and/or its precursor with the ribozyme according to the invention or a mixture thereof.

The agent according to the invention can be used in the combined anti-cancer therapy. In this case, the agent according to the invention contains an additional known component promoting the inhibition of cancer progression for simultaneous or subsequent application in an anti-cancer therapy.

The additional component promoting the inhibition of cancer progression may be a known chemotherapeutic agent such as temozolmide or gliadel or a radiotherapeutic agent. With such a therapy, the efficacy is increased and, due to the targeted activity against the cells with elevated miR-21 content, side effects will be reduced. The agent according to the invention may be used in a combined anti-cancer therapy. The agent is generally administered in suitable pharmaceutical forms, wherein the active agent is associated with a therapeutically allowable carrier. The selection of the carrier will depend on the route of administration and the requirement for protecting it against inactivation or degradation before introducing it or during its introduction to cells, tissues or the organism.

For example, an active agent in the form of nucleic acids can be introduced in liposome systems, preferably those recognizing appropriate type of cells or tissues.

The dosage is determined according to the route of administration, the state requiring the treatment or prevention, as well as other specific conditions.

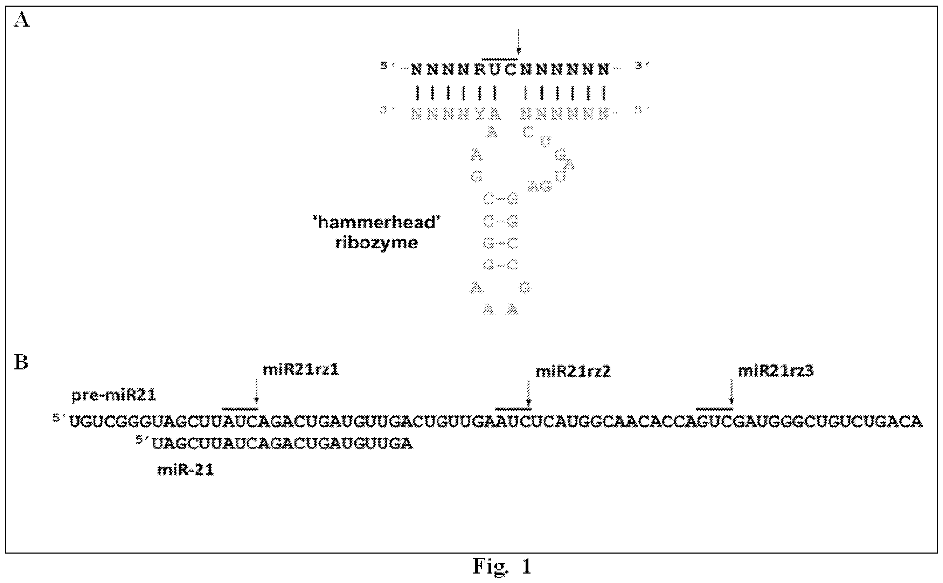

The secondary structure of the hammerhead ribozyme according to the invention is shown in **Fig.1****.** Hammerhead ribozyme according to the invention consists of a catalytic core with a sequence of SEQ ID No 1 and arms complementary to the region within miR-21 and/or its precursor. Preferably, the arms are 6 nucleotides in length. The complementarity of the ribozyme arms to the substrate is the required condition for cleavage. This guarantees the high activity, specificity and precision of the designed tool working at *in vitro* conditions, at physiological concentration of Mg²⁺ ions and in the living cells. The length of the ribozyme arms is a compromise between the ribozyme activity and its specificity.

In the preferred ribozyme, the 6 nucleotides-long flanking arms are complementary to a region within the sequence of the mature miR-21 and/or its precursors. The preferred ribozyme is the miR21rz1 with a sequence of SEQ ID No 2, containing a catalytic core with a sequence of SEQ ID No 1. This ribozyme cleaves the chemical bound at the 3'end cytosine in the AUC sequence.

Another preferred ribozyme is the miR21rz2 with a sequence of SEQ ID No 3, containing a catalytic core with a sequence of SEQ ID No 1, cleaving the chemical bound at the 3'end cytosine in the AUC sequence.

A preferred ribozyme is also the miR21rz3 with a sequence of SEQ ID No 4, containing a catalytic core with a sequence of SEQ ID No 1, cleaving the chemical bound at 3'end cytosine in the GUC sequence.

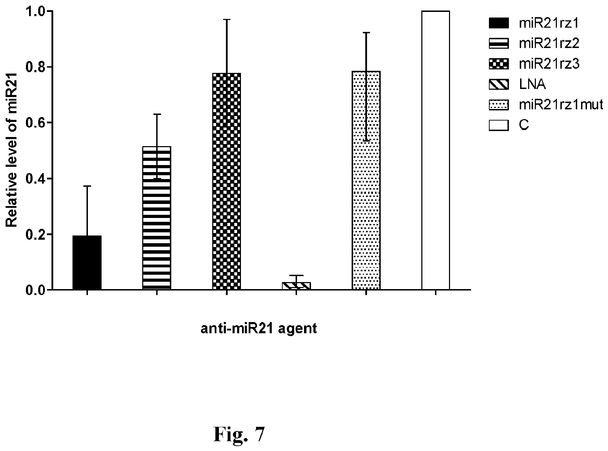

The preferred ribozymes according to the invention demonstrate an activity down regulating the endogenous pool of miR-21 and/or its precursors in the glial cells **(****Fig. 7****).**

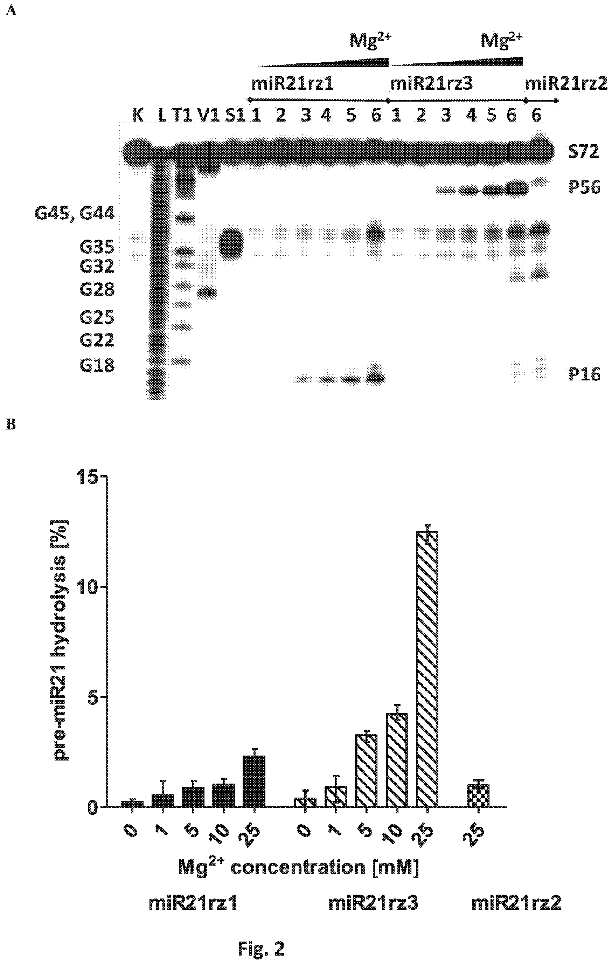

The efficiency of pre-miR-21 cleavage catalyzed by the ribozymes according to the invention depends on Mg²⁺ concentration and occurs already at the physiological concentration of 1 mM, preferably up to 5 mM, more preferably up to 10 mM, the most preferably at 25 mM Mg²⁺ concentration **(****Fig 2A and 2B****).** Ribozymes present in the state of the art, described in Suryawanshi H et al, Mol BioSyst 6, 1807-1809 (2010), in *in vitro* tests exhibit an activity at 25 mM MgCl₂ only (unphysiological conditions). It has not been shown whether the ribozymes exhibit an activity at physiological Mg²⁺ concentration (about 1mM).

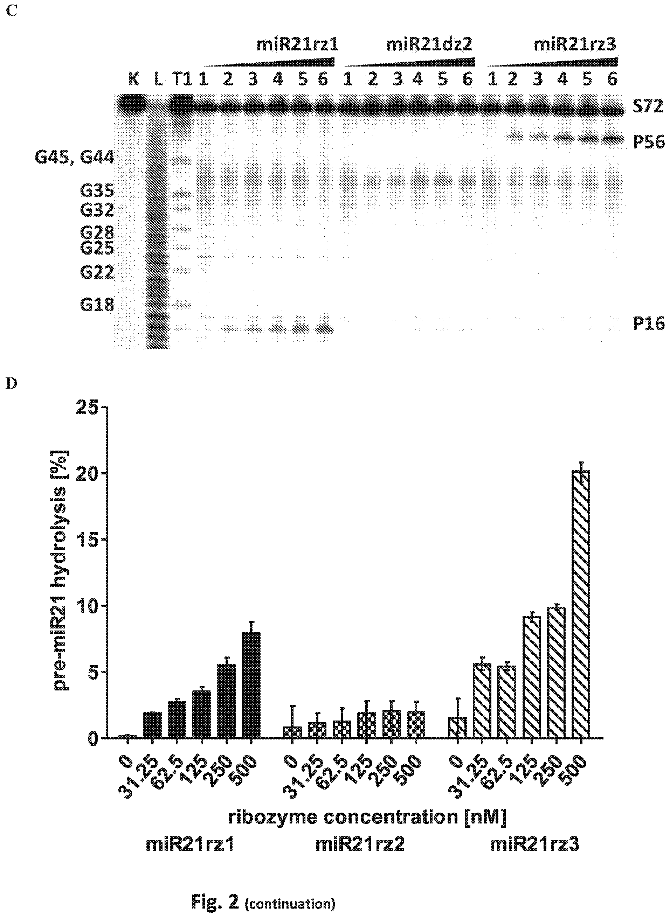

The cleavage efficiency of the pre-miR-21 catalyzed by the ribozymes according to the invention also depends on the ribozymes concentration, increasing with the ribozyme:substrate ratio. The pre-miR-21 cleavage preferably occurs already at 3-fold excess of a ribozyme over substrate, more preferably at 6-fold excess of a ribozyme over substrate, more preferably at 25-fold excess of a ribozyme over substrate, the most preferably at 50-fold excess of a ribozyme over substrate **(****Fig 2C, 2D****,** **4** **and** **5****).**

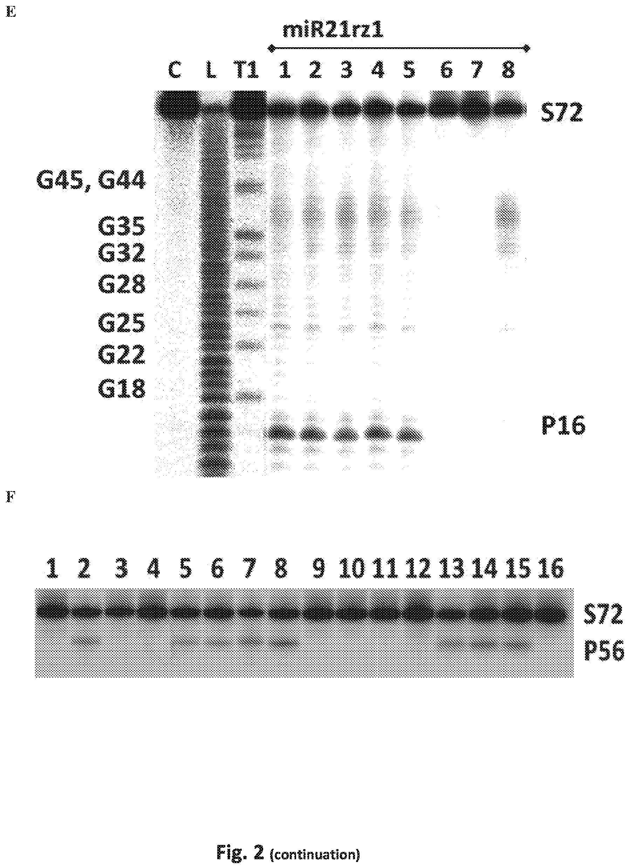

The cleavage efficiency of pre-miR-21 catalyzed by the ribozymes according to the invention requires the Mg²⁺ions, the cleavage does not take place in the presence of monovalent ions (Na⁺, NH₄⁺, Li⁺). The reaction is also inhibited by polyethylene oxides, low-weight (200 and 400) polymers from the polyether's group. The reaction inhibition was not observed in the presence of the other compounds mimicking molecular crowding, such as PEG3350 and 5% MPD **(****Fig. 2F****).** The cleavage efficiency of pre-miR-21 catalyzed by the ribozymes according to the invention is not significantly influenced by the denaturation/renaturation conditions nor by the moment of reaction initiation in the presence of Mg²⁺ **(****Fig. 2E****).**

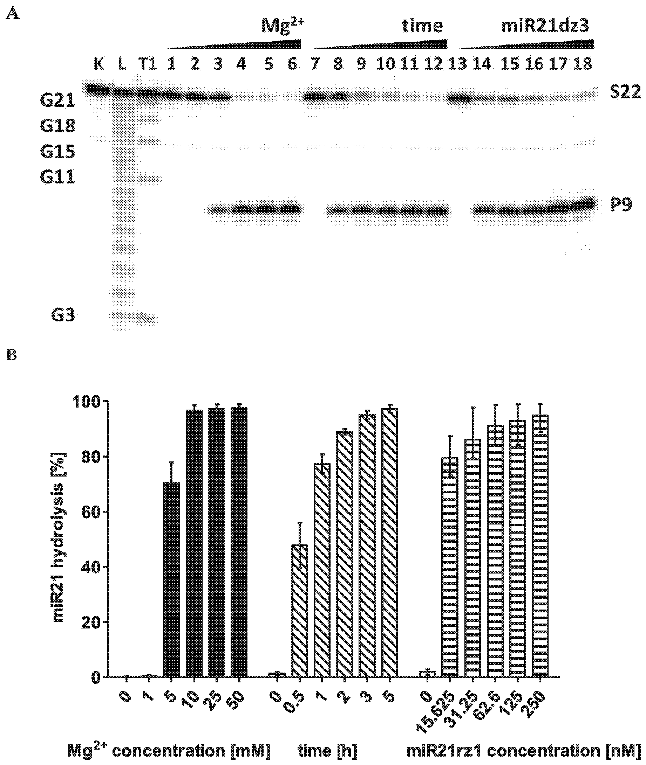

The cleavage efficiency of -miR-21 catalyzed by the ribozymes according to the invention, preferably the ribozyme with a sequence of SEQ ID No 2 depends on concentration of Mg²⁺ ions and proceeds at the 10-fold excess of a ribozyme over substrate in up to 5 mM, preferably up to 10 mM, the most preferably at 25 mM concentration of Mg²⁺ ions **(****Fig. 3A and 3B****).**

The cleavage efficiency of miR-21 catalyzed by the ribozymes according to the invention depends on the ribozymes concentration, increasing with the ribozyme:substrate ratio. The miR-21 cleavage preferably occurs already at 1.5-fold excess of a ribozyme over substrate, more preferably at 3-fold excess of a ribozyme over substrate, more preferably at 6-fold excess of a ribozyme over substrate, more preferably at 25-fold excess of a ribozyme over substrate, the most preferably at 50-fold excess of a ribozyme over substrate **(****Fig. 3A and 3B****).**

The ribozymes according to the invention are characterized by the high activity relative to miR-21. The cleavage efficiency of -miR-21 catalyzed by a ribozyme according to the invention depends on the reaction duration and is efficient already up to 30 minutes, preferably up to 1 hour, more preferably up to 2 hours, more preferably up to 3 hours, the most preferably up to 5 hours **(****Fig. 3A and 3B****).**

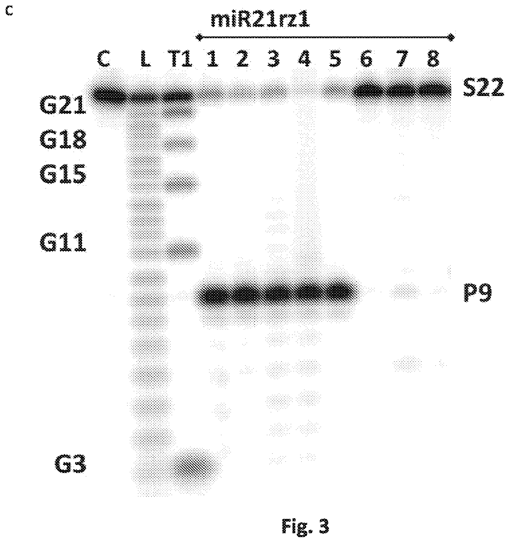

Neither the denaturation/renaturation conditions nor the moment of reaction initiation in the presence of Mg²⁺ ions have no significant impact on miR-21 cleavage efficiency **(****Fig. 3C****).**

In the state of the art no ribozyme activity against mature miR-21 has been described. It has only been postulated, based on the ribozyme arms complementarity to miR-21, that they could be exhibiting such activity (Suryawanshi H et al., Mol BioSyst 6, 1807-1809 (2010).

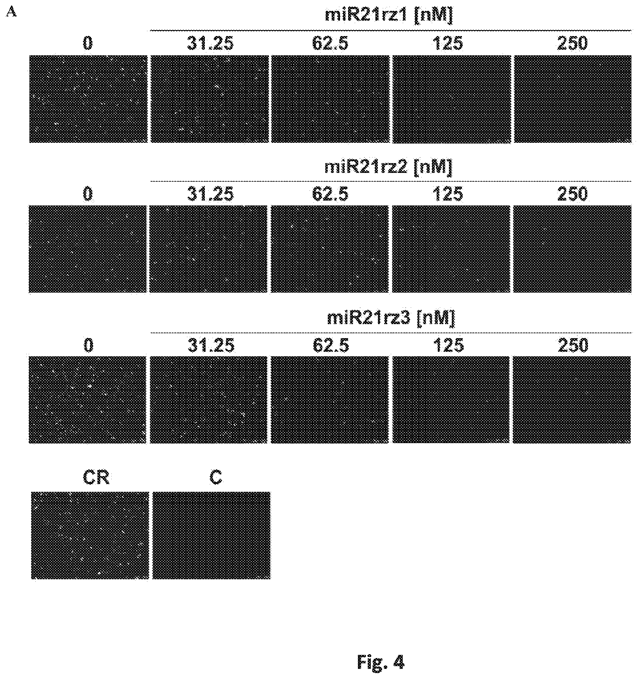

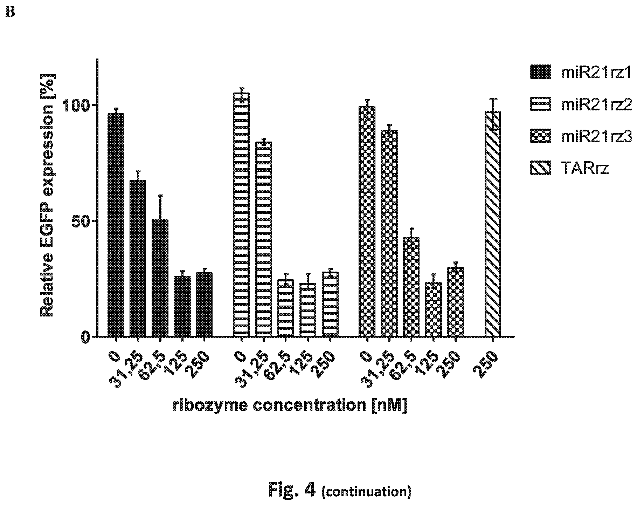

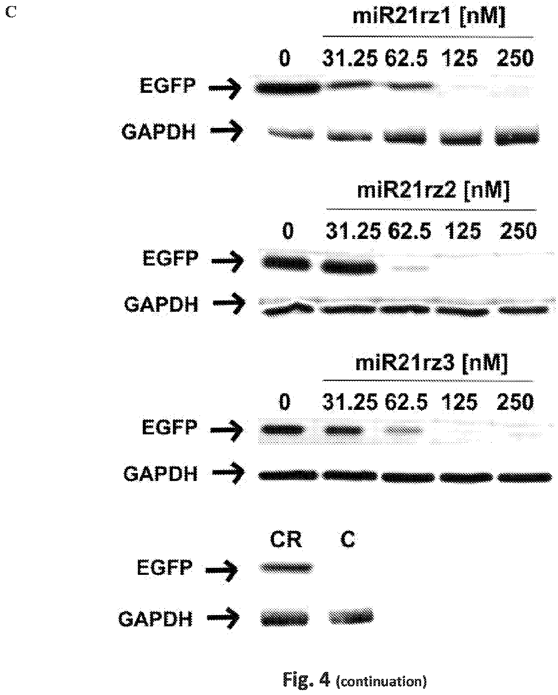

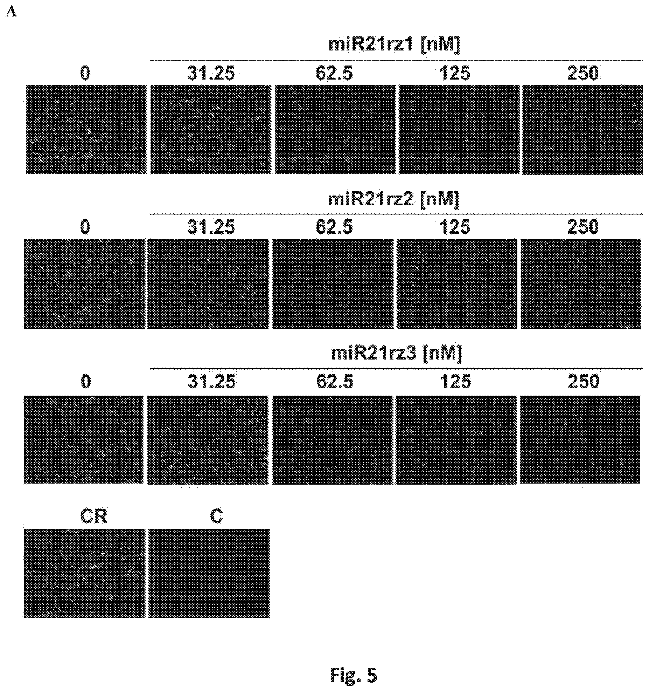

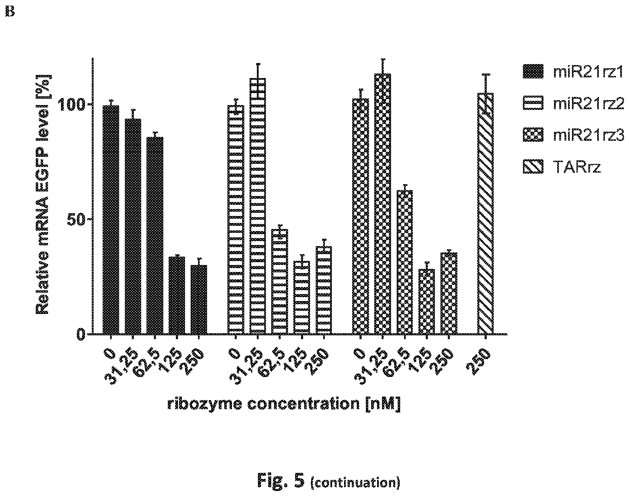

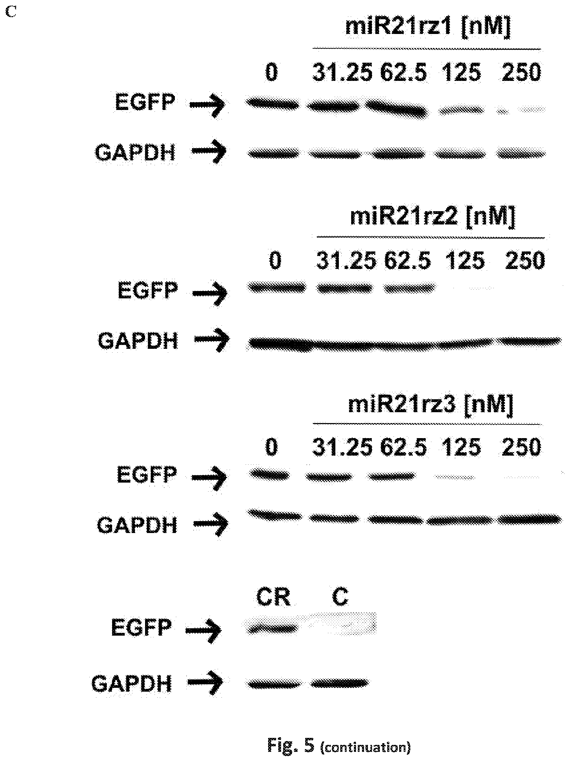

The inventors demonstrated, that the ribozymes are also active in the HeLa and glioblastoma derived T98G cell lines. The cleavage efficiency of pre-miRNA, in the EGFP-based reporter system, catalyzed by the ribozymes according to the invention, depends on the ribozymes types and concentration, increasing with the ribozymes concentration. Pre-miR-21 is cleaved preferably already at the 31.25 nM, more preferably at the 62.5 nM, more preferably at 125 nM, the most preferably at the 250 nM ribozymes concentration in the culture medium. The pre-miR-21 cleavage efficiency in the reporter system catalyzed by the ribozymes according to the invention is similar for all the designed ribozymes. The reaction preferably proceeds with the use of the miR21rz1 and miR21rz3 ribozymes, the most preferably with the use of the miR21rz2 ribozyme **(****Fig. 4** **and** **5****).**

miR21rz1 is the most efficient inhibitor of endogenous miR-21 in cell lines compared to other anti-miR-21 ribozymes. It exhibited the strongest effect in reducing miRNA levels (∼80%) in glioblastoma T98G cultured cells in comparison to miRNA levels in non-transfected control cells. The second most efficient ribozyme was miR21rz2 which showed ∼50% effectiveness in reducing miR-21 levels, whereas both miR21rz3 and miR21rz1-mut **(SEQ ID No 6)** were minimally efficient (∼20%). Ribozymes described in the state of the art, described by the Suryawanshi H et al, Mol BioSyst 6, 1807-1809 (2010), at 1 µM concentration reduce the levels of endogenous miRNA by 40 and 60% for the wild and modified ribozyme respectively **(****Fig. 7****).**

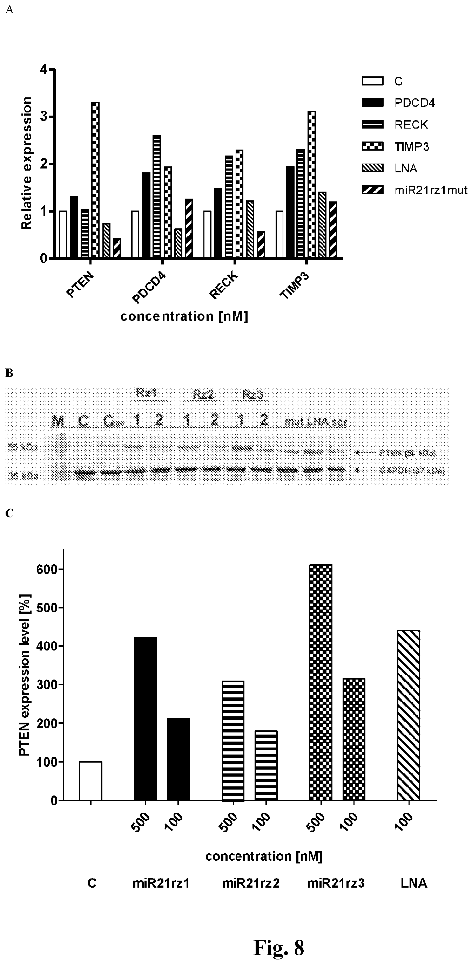

The anti-miR-21 ribozymes are efficient tools for miR-21 silencing in GBM cells, which subsequently significantly influences miR-21 target mRNAs levels. Intracellularly delivered anti-miR-21 ribozymes resulted in the increase of each analyzed target mRNA including PTEN, PDCD4, RECK and TIMP3, wherein miR21rz3 appeared to be the most effective: an over 200% increase in PTEN and TIMP3 expression levels and around 100% increase in RECK and PDCD4 expression levels was observed. A moderate effect was observed in case of miR21rz2 which caused an over 100% increase in PDCD4, TIMP3 and RECK mRNA levels, although it did not affect PTEN. miR21rz1 influenced the expression levels of all analyzed targets inducing a ∼30%, ∼80%, ∼50% and ~90% increase in expression levels of PTEN, PDCD4, RECK and TIMP3, respectively. No significant changes were observed in the case of miR-21rz1-mut transfection, nor did the LNA-transfected cells show any considerable changes in the levels of miR-21 target mRNAs **(****Fig. 8A**).

The upregulation of PTEN in T98G glioma cells after transfection with anti-miR21 ribozymes was observed **(****Fig. 8B****).** The most efficient ribozyme in this regard is miR21rz3, upregulating the PTEN level more than 6 times compared to the control. Scrambled RNAs do not exhibit any effect on the PTEN expression level, which confirms that the effect of anti-miR-21 ribozymes is a specific process **(****Fig. 8B****).**

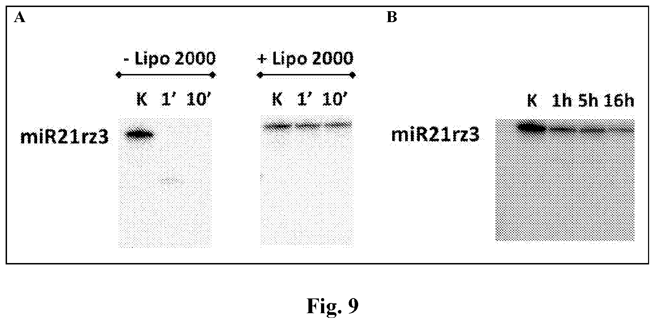

Ribozymes can be unstable and may be readily degraded by the cellular nucleases in physiological conditions. The inventors showed, that the stability of the ribozymes according to the invention, in serum and in the glioblastoma cell lines, is highly improved in the presence of a nucleic acid stability enhancing carrier, preferably Lipofectamine 2000 **(****Fig. 9A and 9B****).**

Publications cited in the description, and the references given therein, are in their entirety incorporated herein as references

### BIREF DECRIPTION OF DRAWINGS

For a better understanding of the invention, it has been illustrated in the embodiments and in the accompanying figures, in which:
**Figure 1** shows secondary structure of the hammerhead ribozyme according to the invention and the cleavages sites within miR-21 and pre-miR-21 for ribozymes directed against pre-mi-R-21/miR-21.
   (A) the sites within miR-21 and pre-miR-21 located within the catalytic core of ribozymes are indicated by lines, cleavage sites are marked by arrows (B). N - any nucleotide, R - purine, U - uridine, A - adenine, C - cytosine, G - guanine.
**Figure 2** shows the results of pre-miR21 cleavage with the use of miR21rz1, miR21rz2 and miR21rz3 (see **Example 2)**
   **Aand B.** Reactions conducted in constant ribozyme concentration (miR21rz1/miR21rz2/miR21rz3) (250 nM) (25-fold ribozyme excess over substrate) and in different Mg²⁺ concentrations (0, 1, 5, 10, 25, 50 mM).
   **C and D.** Reactions conducted in constant Mg²⁺ concentration (10 mM) and different concentrations of ribozymes (miR21rzl/miR21rz2/miR21rz3) (0, 15.625, 31.25, 62.5, 125, 250 nM) (respectively, a 1.5625-, 3.125-, 6.25-, 12.5-, 25-fold ribozyme excess in relation to substrate (pre-miR21)).
   **E.** Reactions conducted in various conditions of denaturation/renaturation and reaction initiation.
   **F.** Reactions conducted in presence of different monovalent and divalent ions and compounds imitating cellular crowding.

   **C -** reaction control; L - OH ladder (50mM NaOH, 10mM EDTA, 95°, 2 min); T1 - limited hydrolysis with RNase T1 (20nM CH₃COONa pH 4.5, 7M urea, 1mM EDTA, 0.025u/µl RNase T1, 55°C, 20 min), V1 - limited hydrolysis with RNase VI (50mM TrisHCl pH 7.5, 200mM NaCl, 20mM MgCl₂, 0.0002u/µl RNase V1, 25°C, 15min), S1 - limited hydrolysis with nuclease S1 (150mM CH₃COONa pH 8.0, 1M NaCl, 15mM ZnSO₄, 0.0095u/µl nuclease S1, 37°C, 30min), S72 - 72nt reaction substrate (pre-miR21), P56, P16 - 56 and 16 nt products respectively of pre-miR21 hydrolysis with ribozymes,, G45, G44, G35, G32, G28, G25, G22, G18 - products of pre-mR21 hydrolysis with RNase T1, the figures indicate the length of the product.
**Figure 3** shows the results of miR-21 cleavage with miR21rz1 ribozyme (see **Example 2)**
   **A and B.** Reactions conducted in constant miR-21 and different ribozyme (miR21rz1) or MgCl₂ concentrations.
      1-6. Reactions conducted in constant ribozyme (100 nM) concentration (10-fold excess of ribozyme over substrate). Reactions were supplemented with different amounts of Mg²⁺ (to the final concentration of 0, 1, 5, 10, 25, 50 mM), then were incubated in 37°C for 1 hour.
      7-12. Reactions conducted in constant ribozyme concentration (100 nM) (10-fold excess of ribozyme over substrate) and Mg²⁺ concentration (10 mM), for 0.5, 1, 2, 3, 5 hours.
      13-18. Reactions conducted in different concentrations of ribozyme (0, 15.625, 31.25, 62.5, 125, 250 nM) (1.5625-, 3.125-, 6.25-, 12.5-, 25-fold excess of ribozyme over substrate, respectively). Reactions were supplemented with 10 mM Mg²⁺, then incubated for 1 hour in 37°C
   **C.** Reactions conducted in various conditions of denaturation/renaturation and reaction initiation.
      C - reaction control; L - OH ladder (50mM NaOH, 10mM EDTA, 95°, 2 min); T - limited hydrolysis with RNaze T1 (20nM CH₃COONa pH 4.5, 7M urea, 1mM EDTA, 0.025u/µl RNase T1, 55°C, 20 min), S22 - 22 nt substrate (miR21), P9 - 9 nt product of miR-21 cleavage with the ribozyme, G21, G18, G15, G11, G3 - 21-, 18-, 15-, 11-, 3-nt products of miR-21 hydrolysis with RNase T1. The figures indicate the length of the product.
**Figure 4** shows the effect of pre-miR21 cleavage with miR21rz1, miR21rz2 and miR21rz3 ribozymes in an EGFP-based reporter system in HeLa cell line (see **Example 3).**
   **A**. Leica fluorescent microscope images of HeLa cells at 24 h after co-transfection with pEGFP-N3 containing the pre-miR-21 coding sequence and ribozymes at different concentrations.
   **B.** Diagram showing the level of EGFP protein in dependence of different ribozyme concentrations.
   **C.** Western blot analysis showing the level of EGFP protein level in dependence of different ribozyme concentrations.

   **C -** reaction control (cells treated only with Lipofectamine 2000), CR - cells transfected with control ribozyme (TARrz).
**Figure 5** shows the effect of pre-miR21 cleavage with miR21rz1, miR21rz2 and miR21rz3 ribozymes in an EGFP-based reporter system in T98G cell line (see **Example 3).**
   **A**. Leica fluorescent microscope images taken at 24 h after co-transfection with pEGFP-N3 containing the pre-miR21 coding sequence and ribozymes at different concentrations.
   **B.** Diagram showing the level of EGFP protein in dependence of different ribozyme concentrations.
   C. Western blot analysis showing the level of EGFP protein level in dependence of different ribozyme concentrations.

   C - reaction control (cells treated only with Lipofectamine 2000), CR - cells transfected with control ribozyme (TARrz).

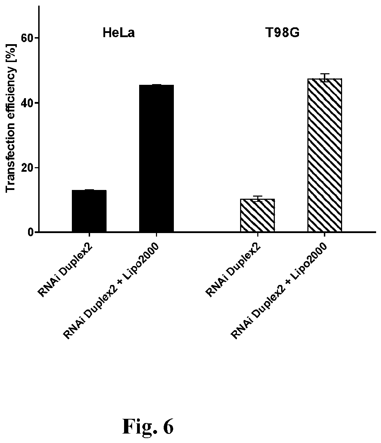

**Figure 6** shows a comparison of transfection efficiency of HeLa and T98G cell lines with 5'-fluorescein-labelled dsRNA (see **Example 3).**
**Figure 7** shows the effect of anti-miR21 ribozymes on the endogenous miR-21 pool in T98G cells (see **Example 4).**
**Figure 8** shows the effect of anti-miR21 agents on relative mRNA levels of miR-21 targets:
   PTEN, PDCD4, RECK and TIMP3 in U118-MG glioblastoma cell line (A) and PTEN protein level in T98G glioblastoma cell line (B) (see **Example 5).**
**Figure 9** shows the results of miR21rz3 stability analysis in human serum (A) and in T98G cells (B) (see **Example 6).**

### DESCRIPTION OF EMBODIMENTS

The following examples are presented merely to illustrate the invention and to clarify its various aspects, but are not intended to be limitative, and should not be equated with all its scope, which is defined in the appended claims.

### EXAMPLES

### EXAMPLE 1

### Production of miR21rz1, miR21rz2, miR21rz3 ribozymes

The hammerhead ribozymes (miR21rz1, miR21rz2, miR21rz3) were designed to target miR-21 and/or pre-miR21 for cleavage. The ribozymes were comprised of 34 nucleotides, including the 22 nucleotide catalytic core with a sequence of SEQ ID No 1 and the flanking 3' and 5' hybridizing arms, preferably six-nucleotide in length, with sequences complementary to miR21 and/or pre-miR21. The miR21rz1 of SEQ ID No 2, miR21rz2 of SEQ ID No 3, miR21rz3 of SEQ ID No 4 were designed and synthetized. The nucleotide catalytic core has been chosen based on inventors' previous studies (Fedoruk-Wyszomirska A et al., J Biochem 145, 451-459 (2009)). The length of flanking arms is the compromise between ribozyme activity and specificity (Kurreck J et al., J Biol Chem 277, 7099-7107 (2000)). The cleavage site has the sequence AUC for ribozymes miR21rz1 and miR21rz2 and GUC for ribozyme miR21rz-3. In all cases, the cleavage occurs on the 3' site of C. The occurrence of these sequences within miR21 and pre-miR21 and the complementarity of their nucleotide sequence to the flanking arms of ribozymes are the relevant elements for the occurrence of cleavage. This provides the high activity, specificity and precision of cleavage of the produced hammerhead ribozymes (miR21rz1, miR21rz2, miR21rz3) when use *in vitro,* at physiological concentrations of magnesium ions and in natural cell conditions.

The secondary structure of hammerhead ribozymes and the chosen target sites for cleavage within pre-miRNA21 and miR-21 sequences are shown in **Fig 1**. The ribozyme miR21rz1 could simultaneously cleave both mature and precursor miRNA.

All the ribozymes were synthesized following standard procedures of RNA synthesis and PAGE-purified by IBA company.

### EXAMPLE 2

### Pre-miR21 and miR-21 cleavage by ribozymes in vitro

The efficiency of pre-miR-21 and miR-21 cleavage by ribozymes *in vitro* was determined at different Mg²⁺ concentrations (0, 1, 5, 10, 25, 50 mM) and substrat:ribozyme ratios **(****Fig. 2** **and** **3****).** The activities of the ribozymes with [³²P] 5'-labeled targets (pre-miR21 and miR21) were measured in 10 µl reaction volumes containing 50 mM Tris-HCl buffer, pH 7.5, at 37 °C. 0, 15.625, 31.25, 62.5, 125, 250 nM ribozyme (respectively, 1.5625-, 3.125-, 6.25-, 12.5-, 25-excess of ribozyme over RNA target). The RNA substrate and ribozymes were denatured for 3 min at 85 °C and cooled down in a heating block to 37 °C at a rate of 0.5 °C/min. Cleavage reactions were carried out at 0, 1, 5, 10, 25, 50 mM MgCl₂ concentrations, for 0-16 hours. The reactions were stopped by adding 10 µl stopping solution (7M urea, 20 mM EDTA, 0.1% bromophenol blue and 0.1% xylene cyanol). Cleavage products were separated by 20% polyacrylamide gel electrophoresis (PAGE) in the presence of 7 M urea in 0.09 M Tris-borate buffer at pH 8.3. The cleavage efficiency was measured based on the product:substrate ratio.

pre-miR-21 is cleaved by miR21rz1, miR21rz2, miR21rz3 ribozymes with different efficiencies. miR21rz3 ribozyme is the most active one. In a 25-excess of miR21rz3 over pre-miR-21, at 25 mM Mg²⁺, after 15 hours 12.5% of substrate was cleaved, 5.5-times more than in the case of miR21rz1. miR21rz2 ribozyme is inactive in any of tested in vitro conditions, however it cleaves the substrate in in vivo conditions. The efficiency of ribozymes depends on the Mg²⁺ concentration and is the highest at 25 mM Mg²⁺, however the ribozymes cleave the substrate even at 1 mM Mg²⁺. The ribozymes described in the state of the art by Suryawanshi H (Suryawanshi H et al., Mol BioSyst 6, 1807-1809 (2010)) cleave the substrate at 25 mM Mg²⁺ (the Mg²⁺ concentration much above physiological conditions). Their activity was not demonstrated at physiological Mg²⁺ concentrations (1mM). The ribozymes cleavage efficiency also depends on the ribozyme:substrate ratio. High ribozyme:substrate ratio results in high ribozyme efficiency. At 10 mM Mg²⁺ and 3-fold excess of ribozyme over substrate, the pre-miR-21 cleavage efficiency is 2% and 3%, at 50-fold excess 9% and 20% respectively for miR21rz1 and miR21rz3 **(****Fig. 2****).**

The effect of monovalent ions and molecular crowding on ribozymes efficiency was tested **(****Fig. 2F****).** The reactions were carried out at constant 10 nM pre-miR-21 in presence of 30 000 cpm [³²P]pre-miR-21, 250 nM miR21rz3 ribozyme, 50 mM TrisHCl, pH 7.5 (1-13), 10 mM MgCl₂ (2-8, 13), 10 mM NaCl (10), 10 mM NH₄Cl (11), 10 mM LiCl (12), 16% PEG200 (3), 16% PEG400 (4), 16% PEG 3350 (5), PEG4000 (6), 40 mM spermine (7), 40 mM spermidine (8), 5% MPD, 20 mM sodium cacodylate, pH 5.5, 10 mM LiCl, 20 mM MgCl₂, 10 mM cobalt hexamine (14), 5% MPD, 20 mM sodium cacodylate, pH 5.5, 20 mM LiCl, 10 mM MgCl₂, 10 mM cobalt hexamine (15), 5% MPD, 20 mM sodium cacodylate, pH 5.5, 30 mM LiCl, 10 mM cobalt hexamine (16). The samples were denatured for 3 min at 85 °C and cooled down in a heating block to 37 °C at a rate of ~0.5 °C/1 min. Hydrolysis reactions were carried out 15 for hours. The reactions without Mg²⁺ were treated as negative controls. The reactions were stopped by adding 10 µl stopping solution and RNAs were separated by 20% polyacrylamide gel electrophoresis (PAGE) in the presence of 7 M urea in 0.09 M Tris-borate buffer at pH 8.3.

It was shown that ribozymes require Mg²⁺ for their activity and are inhibited by polyethylene glycols of low molecule weight (200 and 400). The reaction is not inhibited by other tested molecules imitating molecular crowding: 16% PEG3350 and 5% MPD **(****Fig. 2F****).**

Different conditions of denaturation/renaturation and the moment of reaction initiation by Mg²⁺ supplementation have been tested to evaluate their influence on cleavage efficiency. The cleavage efficiency of RNA catalyzed by the ribozymes according to the invention is not significantly influenced by the denaturation/renaturation conditions nor by the moment of reaction initiation in the presence of Mg²⁺ The reactions were carried out with 10 nM pre-miR21 in presence of 30 000 cpm [³²P]pre-miR21, 10 mM Mg²⁺, 50 mM TrisHCl, pH 7.5, in 37 °C, for 15 hours. 1 - pre-miR21 and ribozyme were denatured separately (85°C, 3 min), then cooled to 37°C mixed and supplemented with MgCl₂; 2 - pre-miR21 and ribozyme were denatured together (85°C, 3 min), then cooled to 37°C and supplemented with MgCl₂; 3 - pre-miR21 and ribozyme were denatured separately (85°C, 3 min), then cooled to 37°C mixed and incubated further in 37 °C; 4 - pre-miR21 and ribozyme were denatured together (85°C, 3 min) in the presence of 10 mM MgCl₂, then cooled to 37 °C; 5 and 6 - RNAs were not denatured nor supplemented (5) or not supplemented (6) with MgCl₂; 7- pre-miR21 and ribozyme were denatured together (85°C, 3 min), not supplemented with MgCl₂; 8 - pre-miR21 (without ribozyme) denatured (85°C, 3 min), then cooled to 37 °C and supplemented with MgCl₂.

The reactions were stopped by adding 10 µl stopping solution and RNAs were separated by 20% polyacrylamide gel electrophoresis (PAGE) in the presence of 7 M urea in 0.09 M Tris-borate buffer at pH 8.3.

In all described conditions the efficiency of pre-miR21 cleavage by ribozyme was demonstrated to be on the same level **(****Fig. 2E****).**

The miR21rz1 ribozyme was designed to recognize and cleave both mature miR-21 and its precursors. The efficiency of miR-21 cleavage by ribozymes *in vitro* was analyzed in 10 µl reaction volumes containing 50 mM Tris-HCl buffer, pH 7.5, constant miR21 concentration in presence of [³²P]-labeled miR21 at different concentrations Mg²⁺ or at different concentrations and ribozyme:substrate ratios, at 37 °C. The RNA substrate and ribozyme were denatured for 3 min at 85 °C and cooled down in a heating block to 37 °C at a rate of ~0.5 °C/min. Hydrolysis reactions were supplemented with MgCl₂ and carried out at 37°C. The reactions were stopped by adding 10 µl stopping solution (7M urea, 20 mM EDTA, 0.1% bromophenol blue and 0.1% xylene cyanol). Cleaved products were separated by 20% polyacrylamide gel electrophoresis (PAGE) in the presence of 7 M urea in 0.09 M Tris-borate buffer at pH 8.3. The cleavage efficiency was measured based on the product:substrate ratio **(****Fig. 3A and B).**

*In vitro* miR-21 is cleaved by miR21rz1 much more efficiently than its precursor pre-miR-21. In a 10-fold excess of ribozyme over substrate and 10 mM Mg²⁺ already after 30 min. almost 60 % of miR-21 is cleaved, which is unachievable for pre-miR-21 even after 15 h of reaction. Prolongation of the reaction gives ∼80% and over 90% of hydrolyzed miR-21 after 1 and 2 hours respectively. miR-21 cleavage by miR21rz1 is also dependent on Mg²⁺ and is still taking place in concentrations lower than in the case of pre-miR-21. At 10-fold excess of ribozyme over miR-21, after 1h, at 5 mM Mg²⁺ 70% of miR-21 is hydrolyzed, at 10 mM Mg²⁺ over 90 %. Furthermore, at 1.5 and 3 ribozyme:substrate ratio, at 10 mM Mg²⁺, after 1 h, almost 80% and 90% of miR21 is hydrolyzed respectively. Further increase in ribozyme excess over substrate results in only marginal improvement of cleavage efficiency.

In the state of the art, ribozyme activity against mature miRNA have not been described. Only Suryawanshi and college have postulated, theoretically and based on their ribozyme nucleotide sequence complementarity to mature miR-21, that their ribozyme could also exhibit such activity (Suryawanshi H et al., Mol BioSyst 6, 1807-1809 (2010).

Different conditions of denaturation/renaturation and the moment of reaction initiation by Mg²⁺ supplementation were tested **(****Fig. 3C****),** The reactions were carried out in constant ribozyme miR21rz1 concentration (100 nM), at 10 nM miR-21 in presence of 30 000 cpm [³²P]miR-21, in constant 10 mM Mg²⁺, 50 mM TrisHCl, pH 7.5, in 37 °C, for 5 hours. 1 -miR-21 and ribozyme were denatured separately (85°C, 3 min), then cooled to 37°C, mixed and supplemented with MgCl₂; 2 - miR-21 and ribozyme were denatured together (85°C, 3 min), then cooled to 37°C and supplemented with MgCl₂; 3 - miR-21 and ribozyme were denatured separately (85°C, 3 min) at 10 mM Mg²⁺, then cooled to 37°C, mixed and incubated further in 37 °C; 4 miR-21 and ribozyme were denatured together (85°C, 3 min) at 10 mM MgCl₂, then cooled to 37 °C; 5 and 6 - RNAs were not denatured nor supplemented (5) or not supplemented (6) with MgCl₂; 7- miR-21 and ribozyme were denatured together (85°C, 3 min), not supplemented with MgCl₂; 8 -miR-21 (without ribozyme) denatured (85°C, 3 min), then cooled to 37 °C and supplemented with MgCl₂. The reactions were stopped by adding 10 µl stopping solution (7M urea, 20 mM EDTA, 0.1% bromophenol blue and 0.1% xylene cyanol). Hydrolysis products were separated by 20% polyacrylamide gel electrophoresis (PAGE) in the presence of 7 M urea in 0.09 M Tris-borate buffer at pH 8.3. The cleavage efficiency was measured based on product:substrate ratio.

Similarly to pre-miR-21, miR-21 cleavage efficiency by miR21rz1 does not depend on conditions of denaturation/renaturation and Mg²⁺ supplementation **(****Fig. 3C****).**

### EXAMPLE 3

### Activity of anti-miR-21 ribozymes in vivo in an EGFP-based reporter system in HeLa and glioblastoma derived T98G cell lines

To assess the efficiency of designed ribozymes on pre-miR-21 cleavage in cell cultures, a reporter system based on green fluorescent protein (GFP) was used. pre-miR-21 cDNA sequence was cloned into pEGFP-N3 (BD Biosciences Clontech) in-frame with the EGFR protein, under the control of cytomegalovirus (CMV) promoter. Cell lines were transfected simultaneously with pEGFP-N3 plasmid containing the pre-miR-21 sequence and individual ribozymes directed against pre-miR-21: miR21rz1, miR21rz2 and miR21rz3. Transfection was carried out using Lipofectamine 2000 Transfection Reagent (Invitrogen) according to manufacturer's protocol. The degree of cleavage of the transcript comprising the pre-miR-21 and EGFP mRNA sequence was evaluated 24 hours after transfection by measuring the level of fluorescence and by analysis of the EGFP protein level. Ribozyme-catalyzed hydrolysis of mRNA at the pre-miR-21 fragment prevents protein synthesis of EGFP. As a result, reduced level of EGFP protein and a decrease in fluorescence in ribozyme-treated cells relative to control without ribozyme can be observed.

HeLa and T98G cells were seeded in 24-well plates in RPMI-1640 (Sigma) and EMEM (ATCC) medium, respectively, both supplemented with 10% FBS (Sigma-Aldrich), 1% vitamins and 1% antibiotics (Sigma) and grown under standard growth conditions (37°C, 5% CO₂). After having reached ∼70% confluence, cell cultures were transfected with catalytic RNAs (at 31.25, 62.5, 125 and 250 nM final concentrations) and pEGFP-N3 plasmid encoding the pre-miR21 sequence (0.8 µg/well). Transfection was carried out using Lipofectamine 2000 Transfection Reagent (Invitrogen) according to manufacturer's protocol. Prior to transfection, the cells were washed with PBS buffer and the medium was changed for non-supplemented counterpart. The hydrolysis yield of transcripts containing pre-miR21 sequence and EGFP mRNA through ribozyme cleavage was estimated 24 hours after transfection by: 1) observation of the cell line using a Leica fluorescence microscope **(****Fig. 4A**), 2) fluorescence measurement using the Multi-mode BioTek Microplate Reader Synergy2 **(****Fig. 4B****)** and 3) assessment of EGFP protein levels using Western blot technique **(****Fig. 4C****).**

In Western blot analysis, 30 µg of total protein extracts were separated on a 18% SDS-PAGE in a presence of protein molecular weight marker. After electrophoresis, the proteins were blotted onto a polyvinylidene fluoride (PVDF) membrane (1h, 350 mM, 100V), blocked in 10% non-fat milk for 2 hrs, washed with PBS and PBS containing 0.1% Tween 20 buffers, and finally incubated with anti-GFP or anti-GAPDH primary antibodies (1:500, Santa Cruz Biotechnology) with biotin-labeled secondary antibody (1:1000, Sigma Aldrich) for 2 h at room temperature. The antibodies were diluted in PBS buffer containing 0.1% Tween and 3% BSA. After antibody incubation, the blots were incubated with Streptavidin alkaline phosphatase conjugate (GE Healthcare, 10 µl/5 ml PBS buffer) for 15 min at room temperature and then subjected to immunodetection by incubation in Sigma Fast BCIP/NBT (Sigma Aldrich). Each step was preceded with rinsing the membrane successively in PBS, PBS containing 0.1% Tween 20 and PBS buffers, for 10 minutes for each washing.

Either cell cultures transfected only with pEGFP-N3 plasmid encoding the pre-miR21 sequence or cells treated with the plasmid and a non-specific ribozyme (lack of sequence complementarity for pre-miR-21) served as controls for the experiment.

All tested ribozymes (miR21rz1, miR21rz2, miR21rz3) exhibit similar activity in cell lines **(Table 3,** **Figure 4** **and** **5****),** wherein the activity of ribozymes is slightly higher in the T98G cell line as compared with the HeLa cell line.

**Tab. 3. IC₅₀ values for ribozymes miR21rz1, miR21rz2, miR21rz3 in T98G and HeLa cell lines. IC₅₀ values were obtained by measurement of fluorescence emitted by cell cultures 24 h after transfection with pEGFP-N3 plasmid containing the pre-miR21 sequence. IC₅₀ calculation and statistics were performed in the GraphPadPrism.**

| | miR21rz1 | miR21rz2 | miR21rz3 |
|---|---|---|---|
| IC₅₀ (T98G) | 115.5 nM | 91.2 nM | 99.2 nM |
| IC₅₀ (HeLa) | 60.2 nM | 53.0 nM | 69.2 nM |

In order to verify if observed differences in fluorescence are not due to differences in susceptibility of these cell lines to transfection, HeLa and T98G cell cultures were grown in 24-well plates in RPMI-1640 medium (Sigma Aldrich) supplemented with 10% FBS (Sigma Aldrich), 1% vitamins and 1% antibiotics (Sigma) and EMEM (ATCC) with the same supplements, respectively. Cell lines were cultured at 37°C under a humidified atmosphere with 5% CO₂. After having reached ∼70% confluence, cell cultures were transfected with fluoresceinlabeled dsRNA oligomer at final concentration 30 nM (BLOCK-iT Fluorescent Oligo, Invitrogen). Transfection was carried out using Lipofectamine 2000 Transfection Reagent (Invitrogen) according to manufacturer's protocol. Prior to transfection, the cells were washed with PBS buffer and the medium was changed for a non-supplemented counterpart. The assessment of transfection efficiency was carried out 24 hrs after transfection based on the observation of the cell line using a Leica fluorescence microscope (A) and fluorescence measurement using Multi-mode BioTek Microplate Reader Synergy2 (B). The efficiency of transfection was estimated with or without Lipofectamine 2000 presence.

The transfection efficiency of fluorescent dsRNA duplexes was comparable in both cases (~45%), with a small predominance (2%) of T98G cell line **(****Fig. 6****).** In both cell lines, the best activity was observed in case of miR21rz2, IC₅₀ = 91.2 nM and =53 nM, respectively for T98G and HeLa cells. In applied conditions, the maximum degree of EGFP silencing is similar for all ribozymes and accounts for 76% and 69% in HeLa and T98G cell line, respectively. It is postulated that the differences in the activity of ribozymes in different human cell lines may be due to different amount of endogenous miR-21 and pre-miR-21 pools in these lines. T98G cells have a much higher level of endogenous pre-miR-21 and miR-21 than the HeLa cell line. One can speculate that ribozymes delivered to the cell cultures are involved in the hydrolysis of both endogenous pre-miR-21 and mature miR-21. With a high endogenous level of miR-21 and pre-miR-21, a decrease in both fluorescence and EGFP protein level, does not fully reflect a degree of hydrolysis of pre-miR-21 in the cells. It only shows the degree of hydrolysis of transcripts containing pre-miR-21 sequence conjugated with EGFP mRNA.

### EXAMLPE 4

### Activity of anti-miR-21 ribozymes in T98G glioblastoma cell line - an influence of anti-miR-21 ribozymes on endogenous level of miR-21 in T98G cells

The influence of anti-miR-21 ribozymes on endogenous expression of miR-21 was analyzed using established glioblastoma cell line purchased from American Type Culture Collection (ATCC) - T98G. T98G cells were grown on ATCC-formulated EMEM medium supplemented with 10% fetal bovine serum (FBS, Sigma Aldrich) and 1x antibiotics (Penicillin-Streptomycin Solution, ATCC) in 75 cm² flasks in standard conditions (37°C, 5% CO₂). 50x10³ of cells were seeded on 24-well plates and after having reached ~80% confluence, the medium was changed for a non-supplemented counterpart. Further, T98G cells were transfected using Lipofectamine 2000 Transfection Reagent (Invitogen) with: anti-miR21 ribozymes (miR21rz1, miR21rz2 and miR21rz3 100 nM each), anti-miR21rz1 mutated in the catalytic core (SEQ ID No 1) (further as miR-21rz1mut of SEQ ID No 6, 100 nM) and antisense anti-miR21 antagomir with LNA modifications purchased from Exiqon (LNA, 50 nM). Cells treated with LNA antagomir were used as positive control, whereas non-transfected T98G cells treated only with Lipofectamine 2000 served as negative control.

24 h post-transfection cells were harvested using TriPure Isolation Reagent (Roche) and used for total RNA isolation according to manufacturer's protocol. RNA samples were treated with DNase I using DNA-free DNase Treatement and Removal Reagent (Ambion) and assessed in terms of quantity and quality using Agilent 2100 Bioanalyzer and RNA 6000 Nano Kit (Agilent Technologies) and NanoDrop 2000 Spectrophotometer (Thermo Scientific). 300 ng of each RNA sample was polyadenylated and reverse-transcribed using miRNA 1^{st}-Strand cDNA Synthesis Kit (Agilent Technologies). The cDNA was further diluted 1:2 with RNase-free water prior to quantification by qRT-PCR. Relative expression of miR-21 was quantified using miR-21 forward primer (Agilent Technologies) and Universal Reverse Primer (Agilent Technologies) in quantitative RT-PCR (real-time PCR). qRT-PCR reactions were conducted using LightCycler 480 System (Roche). miR-21 expression was normalized using 18S rRNA as reference and E-Method for calculation of relative expression of miR-21. The results are shown in **Figure 7****.** Results are means±S.D. from three independent experiments. They showed that miR21rz1 is the most efficient inhibitor of endogenous miR-21 in cell lines comparing to other anti-miR21 ribozymes. It exhibited the strongest effect on reduction miRNA level (~80%) in glioblastoma T98G cultured cells in comparison to miRNA level in non-transfected control cells. The second most efficient ribozyme was miR21rz2 which showed ∼50% effectiveness in reduction of miR-21 expression level, whereas both miR21rz3 and miR21rz1-mut were less efficient (∼20%).

Ribozymes being the state of the art, described by the Suryawanshi H et al, Mol BioSyst 6, 1807-1809 (2010), at 1 µM concentration reduce the level of endogenous miRNA by 40 and 60% for the wild and modified ribozyme respectively.

### EXAMLPE 5

### Activity of anti-miR-21 ribozymes in U118-MG glioblastoma cell line towards miR-21 targets

To determine the effect of anti-miR-21 ribozymes on the level of previously assessed miR-21 targets, we performed transfection experiments in U118-MG glioblastoma cell line and evaluated the levels of four mRNAs using quantitative RT-PCR. Altogether, four different miR-21 targets were analyzed: PTEN (phosphatase and tensin homolog deleted on chromosome ten), PDCD4 (Programmed cell death 4), RECK (reversion-inducing-cysteine-rich protein with kazal motifs) and TIMP3 (Tissue inhibitor of metalloproteinases-3).

U118-MG glioblastoma cell line was purchased from American Type Culture Collection (ATCC). The cells were grown on ATCC-formulated DMEM medium supplemented with 10% fetal bovine serum (FBS, Sigma Aldrich) and 1x antibiotics (Penicillin-Streptomycin Solution, ATCC) in 75 cm² flasks in standard conditions (37°C, 5% CO₂). 50x10³ of cells were seeded on 24-well plates and after having reached ∼80% confluence, the medium was changed for a non-supplemented counterpart. Further, U118-MG cells were transfected using Lipofectamine 2000 Transfection Reagent (Invitogen) with: anti-miR-21 ribozymes (miR21rz1, miR21rz2 and miR21rz3 150 nM each), miR21rz1 mutated in the catalytic core (miR-21rz1-mut, 100 nM) and antisense anti-miR-21 antagomir with LNA modifications purchased from Exiqon (LNA, 50 nM). Cells treated with LNA antagomir were used as positive control, whereas non-transfected T98G cells treated only with Lipofectamine 2000 served as negative control.

48 h post-transfection cells were harvested using TriPure Isolation Reagent (Roche) and used for total RNA isolation according to manufacturer's protocol. RNA samples were treated with DNase I using DNA-free DNase Treatement and Removal Reagent (Ambion) and assessed in terms of quantity and quality using Agilent 2100 Bioanalyzer and RNA 6000 Nano Kit (Agilent Technologies) and NanoDrop 2000 Spectrophotometer (Thermo Scientific). 300 ng of each RNA sample was reverse-transcribed using RevertAid H Minus First Strand cDNA Synthesis Kit (Fermentas). The cDNA was further diluted 1:2 with RNase-free water prior to quantification by qPCR. Relative expression of each target mRNAs was determined in quantitative RT-PCRs (qRT-PCRs, real-time PCRs) which were carried out using specific primers (Genomed), LightCycler 480 Masters Probes Kit (Roche) and UPL probes (Roche). qRT-PCR reactions were conducted using LightCycler 480 System (Roche). Relative mRNA levels were determined following the E-Method (Roche) for relative mRNA quantification in the presence of target and reference genes with different amplification efficiencies. HPRT was used as a reference gene.

The results showed that anti-miR-21 ribozymes are efficient tools for miR-21 silencing in GBM cells, which subsequently significantly influences the miR-21 target mRNAs levels. Intracellularly delivered anti-miR-21 ribozymes resulted in an increase for each analyzed target mRNA including PTEN, PDCD4, RECK and TIMP3, wherein miR21rz3 appeared to be the most effective: an over 200% increase in PTEN and TIMP3 expression levels and an around 100% increase in RECK and PDCD4 expression levels was observed. A moderate effect was observed in case of miR21rz2 which caused an over 100% increase in PDCD4, TIMP3 and RECK mRNA levels, although it did not affect PTEN. miR21rz1 influenced expression levels of all analyzed targets inducing a ∼30%, ∼80%, ∼50% and ∼90% increase in expression levels of PTEN, PDCD4, RECK and TIMP3, respectively. No significant changes were observed in the case of miR-21rz1-mut transfection, nor did the LNA-transfected cells show any considerable changes in the levels of miR-21 target mRNAs.

The effect of anti-miR-21 ribozymes on PTEN protein (miR-21 target) level in T98G cell line has been also determined.

PTEN is one of the best known and widely studied targets for miR-21. When PTEN enzyme is functioning properly, it acts as a part of a chemical pathway signaling the cells to stop dividing and it can induce the cells to undergo programmed cell death (apoptosis) when necessary. These functions prevent uncontrolled cell growth that can lead to the formation of tumors. There is also evidence that the protein encoded by PTEN gene may play a role in cell movement (migration) and adhesion of cells to surrounding tissues. Mutations and deletions of PTEN inactivate its enzymatic activity leading to increased cell proliferation and reduced cell death. Frequent genetic inactivation of PTEN occurs in glioblastoma, endometrial cancer, and prostate cancer; and reduced expression is found in many other tumor types such as lung and breast cancer.

To test the activity of anti-miR-21 ribozymes, Western blot analysis prior to detection of PTEN expression level was performed.
T98G cell line was cultured on 6-well plates in EMEM (ATCC) supplemented with 10% FBS (Sigma Aldrich), 1% antibiotic-antimycotic (Sigma) and 1% vitamins solution (Sigma). The cells were transfected in the presence of Lipofectamine 2000 (Invitrogen) with anti-miR-21 ribozymes (100 and 500 nM), an anti-miRNA ribozyme mutated in the catalytical core (negative control), a scrambled, unspecific oligonucleotide and an oligonucleotide fully complementary to mature miR-21 with LNA modifications (antagomir, positive control). After 48 hrs the cells were harvested and suspended in 10 miM Tris, pH 7,5 buffer containing 10% protease inhibitors (Roche). Cells were sonicated prior to protein isolation, then centrifuged, supernatant was removed and was measured in respect of protein concentration.

50 µg of protein extracts from the T98G cell line were separated on 15% SDS-PAGE. Proteins were blotted for 45 min at 100V, 130 mA at RT in wet blotter (BioRad) onto PVDF membrane 0.45 µm pore size in transfer buffer (25 mM Tris base, 190 mM glycine, 20% methanol). Blots were rinsed once in PBST₂₀ (PBS, 0.05% Tween 20). Membranes were blocked, then hybridized with PTEN specific monoclonal (A2B1) (Santa Cruz Biotechnology, INC) and anti-mouse IgG - biotinylated (Sigma) antibodies with SNAP Protein Detection System (Millipore) according to manufacturer's instructions. Detection of protein was carried out with streptavidin alkaline phosphatase conjugate (Amersham Biosciences) and FAST™ BCIP/NBT buffer (Sigma).

We have observed upregulation of PTEN in T98G glioma cells after transfection with anti-miR-21 ribozymes **(****Fig. 8B****).** Using a specific antibody, we noticed that all ribozymes efficiently restored the protein level in comparison to the control. The most efficient ribozyme is miR21rz3, which upregulates the PTEN level more than 6-foldin comparison to the control. Scrambled RNAs do not show any effect on PTEN expression level which confirms that the effect of anti-miR-21 ribozymes is a specific process.

### EXAMPLE 6

### Stability of Hammerhead ribozymes directed against miR-21 and pre-miR-21 in human serum and T98G cell culture

The designed RNA-based catalytic tools aimed at miR-21 and its precursors are intended to be used in the treatment of GBM. Therefore, it was reasonable to determine their stability in an environment reflecting the conditions of the human body. The stability of RNAs was evaluated under different conditions. *In vitro* the experiment involved an analysis of the ribozymes in human serum, whereas under *in vivo* conditions RNAs were investigated in an established glioblastoma cell line, T98G (ATCC) **(****Fig. 9A and B).**

To evaluate the stability of the analyzed RNAs in human serum, both miR21rz3 (100 nM) and radiolabeled [³²P]miR21rz3 were incubated in the serum for 1 or 10 minutes at 37°C. The reactions were performed in 10 µl volume with or without Lipofectamine 2000 Transfection Reagent (Invitrogen), respectively (RNA was mixed with 1 µl of Lipofectamine 2000 before adding the serum). The reactions were stopped by adding a loading dye which contained 7 M urea, 20 mM EDTA, 0.1 % bromophenol blue and 0.1% xylene cyanole. RNA samples were analyzed by 20% denaturing polyacrylamide gel electrophoresis (PAGE with 7M urea).

To assess the *in vivo* stability of ribozymes in a glioblastoma cell culture, the T98G cells were seeded in 24-well plates in ATCC-formulated EMEM medium supplemented with 10% FBS and 1% antibiotics (Penicillin-Streptomycin Solution, ATCC) under standard growth conditions (37°C, 5% CO₂). After having reached ∼70% confluence, T98G cells were transfected with 250 nM miR21rz3 in the presence of 60000 cpm [32P]miR21rz3. The transfection was carried out using Lipofectamine 2000 (Invitrogen) according to manufacturer's protocol. Prior to transfection, the cells were washed with PBS buffer and the medium was changed for a non-supplemented counterpart. After 1, 5 and 16 hrs post-transfection, the cells were harvested using TriPure Isolation Reagent (Roche) and used for total RNA isolation according to manufacturer's protocol. Next, the RNA samples were supplemented with loading dye (7 M urea, 20 mM EDTA, 0.1 % bromophenol blue and 0.1% xylene cyanole) and analyzed by 20% denaturing PAGE with 7M urea.

The results showed that the ribozyme miR21rz3 is unstable in human serum. After 1 minute incubation with the serum it was hydrolyzed in almost 100%. miR21rz3 become more stable when incubated with Lipofectamine 2000. In the presence of that carrier the ribozyme did not undergo degradation in serum even after 10 minutes of incubation. In cell culture conditions (T98G), we did not observe a substantial loss of the ribozyme integrity. Also, in this case, the fact of ribozyme stability in cell culture can be associated with the presence of Lipofectamine 2000 which stabilizes the transfected RNA **(****Fig. 9****).**

In the following sequence listing:
**SEQ ID No 1** - nucleotide sequence of the catalytic core of hammerhead ribozymes, according to the invention 5'- CUGAUGAGGCCGAAAGGCCGAA-3'
**SEQ ID No 2 -** miR21rz1 ribozyme 5'-CAGUCUCUGAUGAGGCCGAAAGGCCGAAAUAAGC-3'
**SEQ ID No 3 -** miR21rz2 ribozyme 5'-CCAUGACUGAUGAGGCCGAAAGGCCGAAAUUCAA-3'
**SEQ ID No 4 -** miR21rz3 ribozyme 5'-CCCAUCCUGAUGAGGCCGAAAGGCCGAAACUGGU-3'
**SEQ ID No 5** - hammerhead ribozyme described in Suryawanshi H et al. Mol Biosystem 6, 1807-1809 (2010)
**SEQ ID No 6 -** miR21rz1-mut ribozyme 5'-CAGUCUCUGAUGAGGCCGAAAGGCCGAAAUAAGC 3'

## Claims

1. A ribozyme directed against the sequence of miR-21 and/or miR-21 precursors, **characterized in that** it has a sequence as shown in SEQ ID NO: 2 with a catalytic core with a sequence as shown in SEQ ID No 1, and having the ability to specifically cleave miR-21 and/or its precursors.

2. A ribozyme directed against the sequence of miR-21 precursors, **characterized in that** it has a sequence as shown in SEQ ID NO: 3 with a catalytic core with a sequence as shown in SEQ ID No 1, and having the ability to specifically cleave miR-21 and/or its precursors.

3. A ribozyme directed against the sequence of miR-21 precursors, **characterized in that** it has a sequence as shown in SEQ ID NO: 4 with a catalytic core with a sequence as shown in SEQ ID No 1, and having the ability to specifically cleave miR-21 and/or its precursors.

4. A composition, **characterized in that** it comprises at least one ribozyme according to any of claims 1-3 or a mixture thereof.

5. The composition according to claim 4, **characterized in that** it comprises a carrier facilitating the transport of ribozymes through cell membranes.

6. A therapeutic agent, **characterized in that** it comprises as an active agent at least one ribozyme as defined in any of claims 1-3 or a mixture thereof.

7. The therapeutic agent according to claim 6, **characterized in that** it also contains a different component for inhibiting tumor cells growth for simultaneous or subsequent use in an anti-cancer therapy.

8. The therapeutic agent according to claim 7, **characterized in that** the different component for inhibiting tumor cells growth is temozolomide or gliadel and wherein the cancer is a brain tumor, preferably a brain glioma, more preferably glioblastoma multiforme.

9. A in vitro method of selective cleavage of miR-21 and/or miR-21 precursors, wherein the method comprises a formation of a complex of the cleavage substrate, which is miR-21 and/or pre-miR-21, with a ribozyme as defined in claims 1-3 or a mixture thereof.

10. A ribozyme as defined in any of claims 1-3 or a mixture thereof, or a composition as defined in claims 4-5 or a therapeutic agent as defined in claims 6-8 for use in the treatment of diseases with elevated cellular miRNA pool for miR-21 and/or miR-21 precursors, preferably cancer with elevated cellular miRNA pool for miR-21 and/or miR-21 precursors, preferably the cancer is a brain tumor, preferably brain glioma, more preferably glioblastoma multiforme.

## Patentansprüche

1. Ein Ribozym, das gegen die Sequenz von miR-21 und/oder miR-21-Vorläufern gerichtet ist, **dadurch gekennzeichnet, dass** es eine Sequenz aufweist, wie in SEQ ID NO: 2 gezeigt wird, mit einer katalytischen Kern mit einer Sequenz wie in SEQ ID No 1 gezeigt wird, und mit der Fähigkeit miR-21 und/oder deren Vorläufern spezifisch zu spalten.

2. Ein Ribozym, das gegen die Sequenz von miR-21-Vorläufern gerichtet ist, **dadurch gekennzeichnet, dass** es eine Sequenz aufweist, wie in SEQ ID NO: 3 gezeigt wird, mit einer katalytischen Kern mit einer Sequenz wie in SEQ ID No 1 gezeigt wird, und mit der Fähigkeit miR-21 und/oder deren Vorläufern spezifisch zu spalten.

3. Ein Ribozym, das gegen die Sequenz von miR-21-Vorläufern gerichtet ist, **dadurch gekennzeichnet, dass** es eine Sequenz aufweist, wie in SEQ ID NO: 4 gezeigt wird, mit einer katalytischen Kern mit einer Sequenz wie in SEQ ID No 1 gezeigt wird, und mit der Fähigkeit miR-21 und/oder deren Vorläufern spezifisch zu spalten.

4. Zusammensetzung, **dadurch gekennzeichnet, dass** sie mindestens ein Ribozym nach einem der Ansprüche 1-3 oder ein Gemisch davon aufweist.

5. Zusammensetzung nach Anspruch 4, **dadurch gekennzeichnet, dass** sie einen Träger umfasst, der den Transport von Ribozymen durch Zellmembranen erleichtert.

6. Therapeutisches Mittel, **dadurch gekennzeichnet, dass** es als Wirkstoff mindestens ein Ribozym, wie es in einem der Ansprüche 1-3 definiert wird, oder ein Gemisch davon aufweist.

7. Therapeutisches Mittel nach Anspruch 6, **dadurch gekennzeichnet, dass** es auch eine andere Komponente zur Hemmung des Wachstums von Tumorzellen für die gleichzeitige oder nachfolgende Verwendung in einer Anti-Krebs-Therapie aufweist.

8. Therapeutisches Mittel nach Anspruch 7, **dadurch gekennzeichnet, dass** die andere Komponente zur Hemmung des Wachstums von Tumorzellen Temozolomid oder Gliadel ist und wobei der Krebs ein Gehirntumor, vorzugsweise ein Gehirngliom, bevorzugter Glioblastoma multiforme ist.

9. Verfahren in vitro zur selektiven Spaltung von miR-21 und/oder miR-21-Vorstufen, wobei das Verfahren die Bildung eines Komplexes des Spaltungssubstrates, das miR-21 und/oder pre-miR-21 ist, mit einem Ribozym, wie in den Ansprüchen 1-3 definiert wird, oder einem Gemisch davon umfasst.

10. Ribozym nach einem der Ansprüche 1-3 oder ein Gemisch davon oder eine Zusammensetzung, wie in Ansprüchen 4-5 definiert wird oder ein therapeutisches Mittel, wie in den Ansprüchen 6-8 definiert wird zur Verwendung bei der Behandlung von Krankheiten mit erhöhtem zellulärem miRNA-Pool für miR-21 und/oder miR-21-Vorläufer, vorzugsweise Krebs mit erhöhtem zellulärem miRNA-Pool für miR-21 und/oder miR-21-Vorläufer, vorzugsweise der Krebs ein Gehirntumor, vorzugsweise Gehirngliom, bevorzugter Glioblastoma multiforme ist.

## Revendications

1. Un ribozyme dirigé contre la séquence de miR-21 et/ou des précurseurs miR-21, **caractérisé en ce qu'**il présente une séquence telle que représentée dans SEQ ID NO: 2 avec un noyau catalytique avec une séquence telle que représentée dans SEQ ID NO: 1, et ayant la capacité de cliver spécifiquement miR-21 et/ou ses précurseurs.

2. Un ribozyme dirigé contre la séquence des précurseurs miR-21, **caractérisé en ce qu'il** présente une séquence telle que représentée dans SEQ ID NO: 3 avec un noyau catalytique avec une séquence telle que représentée dans SEQ ID NO: 1, et ayant la capacité de cliver spécifiquement miR-21 et/ou ses précurseurs.

3. Un ribozyme dirigé contre la séquence des précurseurs miR-21, **caractérisé en ce qu'il** présente une séquence telle que représentée dans SEQ ID NO: 4 avec un noyau catalytique avec une séquence telle que représentée dans SEQ ID NO: 1, et ayant la capacité de cliver spécifiquement miR-21 et/ou ses précurseurs.

4. Une composition, **caractérisée en ce qu**'elle comprend au moins un ribozyme selon l'une quelconque des revendications 1-3 ou un mélange de ceux-ci.

5. La composition selon la revendication 4, **caractérisée en ce qu**'elle comprend un porteur facilitant le transport des ribozymes à travers les membranes cellulaires.

6. Un agent thérapeutique, **caractérisé en ce qu'il** comprend en tant qu'agent actif au moins un ribozyme tel que défini dans l'une quelconque des revendications 1-3 ou un mélange de ceux-ci.

7. L'agent thérapeutique selon la revendication 6, **caractérisé en ce qu'il** contient également un composant différent pour inhiber la croissance des cellules tumorales pour une utilisation simultanée ou ultérieure dans une thérapie anticancéreuse.

8. L'agent thérapeutique selon la revendication 7, **caractérisé en ce que** le composant différent pour inhiber la croissance des cellules tumorales est le temozolomide ou le gliadel et où le cancer est une tumeur cérébrale, de préférence un gliome cérébral, plus préférablement un glioblastome multiforme.

9. Un procédé in vitro de clivage sélectif de miR-21 et/ou des précurseurs miR-21, dans lequel le procédé comprend la formation d'un complexe du substrat de clivage, qui est miR-21 et/ou pre-miR-21, avec un ribozyme tel que défini dans les revendications 1-3 ou un mélange de ceux-ci.

10. Un ribozyme tel que défini dans l'une quelconque des revendications 1-3 ou un mélange de ceux-ci ou une composition telle que définie dans les revendications 4-5 ou un agent thérapeutique tel que défini dans les revendications 6-8 pour utilisation dans le traitement de maladies avec un pool de miARN cellulaire élevé pour miR-21 et/ou précurseurs miR-21, de préférence un cancer avec un pool de miARN cellulaire élevé pour miR-21 et/ou précurseurs miR-21, de préférence le cancer est une tumeur cérébrale, de préférence un gliome cérébral, plus préférablement un glioblastome multiforme.
